# EUROPEAN PATENT APPLICATION

(11) **EP 2 201 976 A1**
(43) Date of publication of application: **30.06.2010**
(21) Application number: 08022330.8
(22) Date of filing: 23.12.2008
(51) Int. Cl.: A61M 5/32

(54) **Apparatus for holding a cover of a needle unit and method**

(71) Applicant: Sanofi-Aventis Deutschland GmbH, 65929 Frankfurt am Main (DE)
(72) Inventor: The designation of the inventor has not yet been filed

(57) **Abstract**

An apparatus comprises a recess configured to hold a cover which is suitable to cover at least a part of a needle unit for a medication delivery device.

## Description

The invention concerns an apparatus for holding a cover of a needle unit and a method for handling the apparatus.

A medication delivery device may have a housing which contains medication. The medication delivery device may comprise a cap which is attachable to a distal part of the housing. The distal part of the housing or the medication contained in the distal part of the housing may be protected by the cap. The cap is detached before medication delivery and attached after medication delivery. A needle unit is attached to the medication delivery device before medication delivery. The needle unit may be detached after medication delivery.

DE 103 27 119 A1 shows a cap for an injection device. The cap serves as attachment tool and/or detachment tool for a needle unit. A recess which is located in the distal end of the cap is formed as attachment/detachment tool.

It is an aim of the present invention to provide an alternative tool for handling a needle unit for a medication delivery device.

For this purpose an apparatus comprises a recess configured to hold a cover which is suitable to cover at least a part of a needle unit for a medication delivery device. The cover is at least partly inserted to the recess. The apparatus serves as tool for holding the cover which may or may not contain a needle unit.

Preferably, the needle unit is attached to the medication delivery device before medication delivery and can be detached after medication delivery. The needle unit is provided in a sterile packaging. The packaging comprises at least one cover which at least partly covers the needle unit. The cover may be removed from the needle unit after attachment and may be reattached to needle unit after medication delivery. The cover shall prevent accidental sticking of the user during the attachment and detachment process. Conventionally, the user uses his or her hand to attach/detach the needle unit and the cover.

One embodiment of the apparatus, which is described here, is configured to attach the cover to the needle unit after medication delivery. The use of the apparatus reduces the risk of sticking injuries because the user's hand is in a safe distance from the needle unit, if the user does not use his or her hand to attach the cover to the sharp needle unit.

Preferably, the apparatus comprises holding means configured to encompass at least a part of the cover in a form-fitting manner so that the cover is located in a predetermined position relative to the apparatus. In one embodiment, the cover, which is encompassed, can rotate. In an alternative embodiment, the cover is encompassed in a rotationally fixed position. More preferably, the apparatus is configured to transmit a transversal and/or a rotational movement of the apparatus to the cover. In other words, the cover can be transversally and/or rotationally moved by means of the apparatus.

The recess of the apparatus may be formed so that the cover can be removed from the apparatus by positioning the apparatus in a vertical direction so that the cap falls out off the recess. Alternatively, the recess may be formed so that the holding means also encompasses the cover in a force-fitting manner or friction-locked manner. The user can remove the cover from the apparatus by shaking out or taking out with one's hand. In the latter case the apparatus is formed so that a part of the cover protrudes from the apparatus if the cover is inserted to the apparatus. The user can grip the protruding part for removing the cover from the apparatus.

One embodiment of the apparatus is configured to attach the needle unit, which is at least partly located in the cover, to the medication delivery device. The needle unit may be attached to the medication delivery device by locating the cover and the needle unit, which is located inside the cover, to the distal end of the medication delivery device, and then moving the apparatus towards the distal end of the medication delivery device so that the needle unit is attached to the medication delivery device. Means for attachment may be designed as snapping means.

In an alternative embodiment, the apparatus serves to screw the needle unit to the distal end of the medication delivery device. In this case the rotational movement of the apparatus is transmitted to the needle unit via the cover.

In one embodiment the cover is encompassed by the recess in a form-fitting manner. The cover is formed to encompass the needle unit in a form-fitting manner, so that the needle unit is screwed to the distal end of the medication delivery device when the user is rotating the apparatus. The apparatus serves as attachment tool which offers the user a safe method for attaching the needle unit. The use of the apparatus reduces the risk of needle sticking injuries because the user does not need to touch the cover or the needle unit.

In one embodiment, the apparatus is configured to place the cover over the needle unit, which is attached to a medication delivery device, and then to detach the needle unit from the medication delivery device. The apparatus serves as detachment tool. If the needle unit is screwed to the distal end of the medication delivery device, the apparatus can be used to unscrew the needle unit.

The above-mentioned cover is preferably designed as an outer cover suitable to cover a needle unit with a needle which can be covered by an inner cover. The outer cover may form a part of the packaging which encases the needle unit. This packaging comprises the outer cover in which the needle unit is located and a seal closing a proximal opening of the cover, so that the packaging provides the needle unit in sterile conditions. Preferably, the needle unit is provided also with an inner cover which covers the distal part of the needle before use.

Before medication delivery the seal is pealed off, and then the needle unit is attached to the medication delivery device preferably without removing the outer and inner covers prior to attachment. Then the outer and the inner covers are removed. After medication delivery the inner and outer covers may be reattached and the needle unit is detached. Some users may reattach only one of the inner and outer covers. Reattaching the covers should prevent needle sticking injuries when the needle unit is being detached and delivered. However, the risk of sticking injuries still exists when the user is reattaching the covers. Even if the inner cover has been attached, the needle may puncture the side wall of the outer cover when the side wall of the outer cover is pushed accidentally to the distal end of the needle.

One embodiment of the apparatus is configured to place the inner cover over the needle of the needle unit after medication delivery. Using the apparatus for attaching the inner cover reduces the risk of needle sticking injuries because the user's hand is in a safe distance from the inner cover and the needle when the apparatus is used as attachment tool for the inner cover.

The risk of needle sticking injuries during attachment of the inner cover is larger than the risk during detachment of the inner cover, because inserting the needle into the inner cover demands greater dexterous abilities. Thus, in one embodiment the user uses his or her hand to remove the inner cover, but the user uses the apparatus to reattach the inner cover to the needle.

Preferably, the recess of the apparatus stores the inner cover after removing it from the needle. For this purpose, a holding means is located in the recess, the holding means being suitable to hold the cover in a predetermined position. In one embodiment the holding means is formed as cavity in the recess having a diameter which corresponds with the diameter of the inner cover. More preferably, the recess is formed to guide the cover to the predetermined position inside the apparatus. The user inserts the inner cover to the recess. The diameter of the opening of the recess is larger than the diameter of the inner cap. Thus, it is easy to insert the inner cover to the opening of the apparatus. In one embodiment the recess is narrowed between the opening and the cavity. Thus the inner cover slides down to the holding means which holds the inner cover in the predetermined position.

The inner cover is attached to the needle when the medication delivery device is inserted to the recess of the apparatus. The recess is formed so that the needle of the needle unit, which is attached to the medication delivery device, is aligned with the inner cover, which is held inside the apparatus. The medication delivery device is guided by the form of the recess so that the inner cover is placed over the needle.

One embodiment of the apparatus is suitable to hold only the outer cover. One embodiment of the apparatus is suitable to hold only the inner cover. A preferred embodiment of the apparatus comprises a recess which is formed so that it is suitable to hold both the outer cover and the inner cover. Alternatively the apparatus comprises two recesses. One is suitable to hold the outer cover and the other one is suitable to hold the inner cover.

Preferably, the apparatus is designed as detachable component of the medication delivery device. More preferably, the apparatus is formed as a cap of a pen-type medication delivery device, wherein the distal part of a housing of the medication delivery device can be inserted to the recess of the cap. If the cap is attached to the housing, the cap protects the distal part of the housing. The cap is detached from the housing before medication delivery and is used as attachment and/or detachment tool.

Preferably the recess of the cap is formed so that the inner cover is placed in a predetermined position inside the cap. When the cap is being attached to the housing, to which the needle unit is attached, the inner cover inside the cap and the needle of the needle unit are aligned. The inner cover is attached to the needle when the cap is placed over the distal part of the housing. Thus, the cap serves as storage and attachment tool for the inner cover. Once the inner cover has been inserted to the cap the inner cover remains in place in the cap and as such, once the medication has been delivered, the inner cover can be replaced safely with considerably low risk of needle sticking injury as a result of the safe distance and enclosure of the needle within the end of the cap. When the inner cover has been attached the tool can be used to detach the needle unit from the medication delivery device.

One embodiment of the apparatus is suitable to attach and/or detach only one of a multitude of needle units or is suitable to attach and/or detach only a group of a multitude of needle units. The tool can be designed for one specific needle whereby the tool may be built exclusively for one medication delivery device design or supplier. The apparatus could be tailored to a particular exclusive needle unit design and/or sharps bin, which could also help with removal of the needle. In other words, one embodiment of the apparatus is usable for only one type of a needle units or a predetermined group of needle units.

A method for using the apparatus comprises inserting a cover into a recess of the apparatus, which is suitable to hold the cover after having been inserted. The cover is suitable to cover at least a part of a needle unit for a medication delivery device. The apparatus may be used to place the inner cover over the needle after medication delivery. Preferably, the cover is attached by means of the apparatus to the needle unit.

The apparatus may be used to attach the needle unit to the medication delivery device. In this case, the needle unit which is at least partly located in the cover is attached to the medication delivery device by means of the apparatus. Detaching the needle unit is performed by placing the cover over the needle unit, and then detaching the needle unit from the medication delivery device. In one embodiment the needle unit is screwed to the medication delivery device when the needle unit is being attached. The needle unit is unscrewed from the medication delivery device when the needle unit is being detached.

Preferably, the cap of the medication delivery device is used as apparatus. If the cap is attached to the medication delivery device, the cap protects the distal part of the medication delivery device. The cap is detached for medication delivery and can be used as attachment and/or detachment tool for the needle unit.

The cap is also used to attach an inner cover suitable to cover the needle of the needle unit. The inner cover is positioned inside the cap. Then the cap is positioned over the distal part of the housing of the medication delivery device. When the distal part of the housing is inserted to the cap, the needle, which is located at the distal end of the housing, is aligned with the inner cover, which is positioned inside the cap, so that the inner cover is placed over the needle.

The apparatus offers the user a safe method for handling a needle unit, e.g. attaching and detaching of the needle unit and attaching the inner cover to the needle. The apparatus prevents direct contact between the needle unit and the user's hand. This reduces the risk of needle sticking injuries. Thus, the tool is of particular benefit to those with dexterity difficulties as it offers the user better purchase at a safe distance from the actual needle.

Other features will become apparent from the following detailed description when considered in conjunction with the accompanying drawings.

Figure 1 shows a section of a needle packaging.

Figure 2 shows a cap of a medication delivery device holding the packaging.

Figure 3 shows the needle unit located inside the cap after peeling off the seal of the packaging.

Figure 4 shows the needle unit attached to a needle hub of the medication delivery device.

Figure 5 shows the attached needle unit after removing an outer cover.

Figure 6 shows a detailed section of the distal part of the cap.

Figure 7 shows a detailed section of the distal part of the cap, wherein an inner cover is held by a holding cavity of the cap.

Figure 8 shows a detailed section of the cap holding the inner cover, the cap being placed over the distal part of the housing.

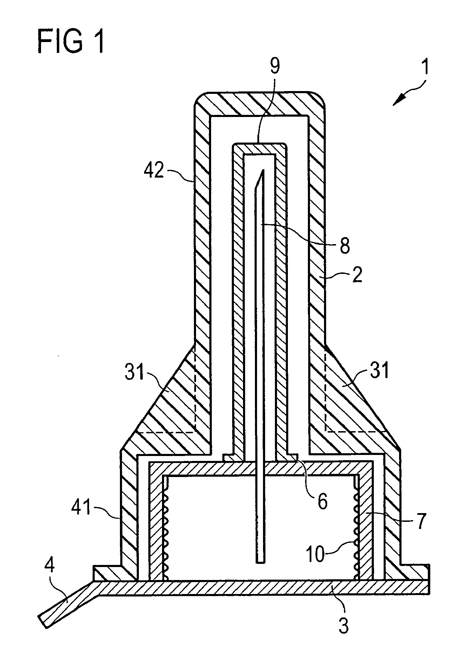

Figure 1 shows a section of a known needle packaging 1. The packaging 1 provides a sterile needle unit 6 for a medication delivery device, the needle unit 6 being located inside the packaging 1.

The needle packaging 1 comprises an outer cover 2 and a seal 3. The outer cover 2 has a proximal opening. A lidding film seals the opening of the outer cover 2. The lidding film forms a seal 3 having a tap 4, which is pulled by a user to tear off the seal 3 before use of the needle unit 6.

The outer cover 2 comprises a proximal part 41 and a distal part 42 which are both formed cylindrically. The diameter of the distal part 42 is smaller than the diameter of the proximal part 41. The distal part 41 is configured to surround a needle 8 of the needle unit 6. The proximal part 42 is configured to surround an attachment 7 of the needle unit 6.

The outer cover 2 comprises first mating means 31. The first mating means 31 are designed as ribs or webs, which are located on the outside wall of the outer cover 2. In this embodiment the ribs are formed triangularly and extend from the top of the proximal part 41 of the outer cover to the side walls of the distal part 42 of the outer cover. The first mating means 1 interact with an apparatus as described later. Conventionally, the ribs are provided to facilitate gripping the outer cover 2.

The needle unit 6 comprises the attachment 7 and the needle 8. The attachment 7 is formed to be releasably connected with a needle hub (13, not shown in figure 1) located at a distal end of a housing of a medication delivery device (12, not shown in figure 1). The attachment 7 comprises an internal thread 10 which matches with an external thread (14, not shown in figure 1) of the needle hub (13, not shown in figure 1). In an alternative embodiment (not shown), the attachment 7 is attached by snapping means. The needle 3 is suitable for delivery of the medication. An inner cover 9 covers the distal part of the needle 3.

The needle unit 6 comprises second mating means (32, not shown in figure 1) which match with third mating means (33, not shown in figure 1) of the outer cover 2 so that the outer cover 2 encompasses at least a part of the needle unit 6 with positive locking. A rotation of the outer cover 2 is transferred to the needle unit 6 by means of the positive connection.

The second and third mating means may be formed as ribs or webs. In one embodiment the second mating means are formed as ribs extending in the axial direction, the ribs being located on the outer side wall of the attachment 7. The third mating means are formed as ribs extending in the axial direction, the ribs being arranged on the inner side wall of the proximal part 41 of the outer cover. If the needle unit 6 is located inside the outer cover 2, one of the ribs of the second mating means may be located between ribs of the third mating means. Preferably, the outer cover 2 also encompasses at least parts of the needle unit 6 in a force-fitting manner, which means that the needle unit 6 is friction-locked in the outer cover 2, so that the needle unit 6 does not fall out off the outer cover 2 after peeling off the seal 3 even if the proximal opening of the outer cover 2 is directed downwards.

The user may peel off the seal 3 and uses the outer cover 2 to locate and attach the needle unit 5. Then the outer cover 2 and the inner cover 9 are removed from the needle unit 6. After medication delivery the user may reattach the inner and/or the outer cover before detaching the needle unit 6 from the medication delivery device. When the user performs these steps he or she would come in contact with the needle 8, which may result in needle sticking injuries. Current needle units 6 rely on a level of dexterity on the part of the user when attaching the needle unit 6 and aligning the inner cover 9 and the needle 8 in order to reattach the inner cover 9 before removing the needle unit 6 from the device.

An apparatus or tool for handling the needle unit 6 improves the safety and ease of attachment and removal of the needle unit 6 from the medication delivery device. It is the removal process that would benefit significantly from the improvement in safety. The attachment and the detachment processes would benefit from the improvement of grip and purchase on the needle unit 6.

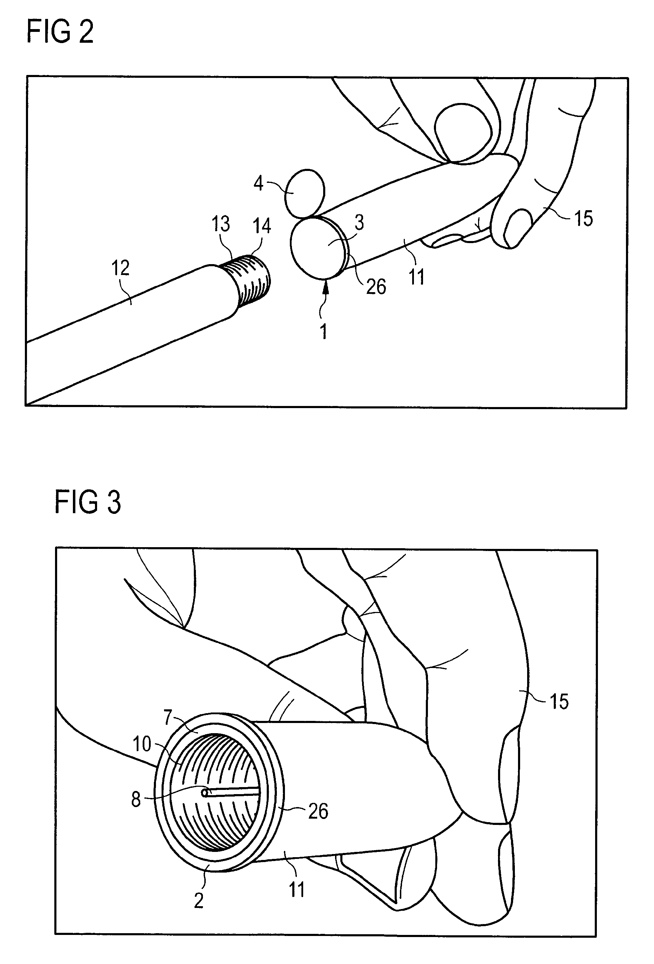

Figure 2 shows a cap 11 of a medication delivery device, the cap 11 serving as apparatus for handling. The cap 2 holds the needle packaging 1 before the needle unit (6, not visible in figure 2) is attached.

The medication delivery device, which comprises a housing 12 and the cap 11, is formed as pen-type delivery device. A distal part of the housing 12 is configured to contain medication. In one embodiment the distal part serves as cartridge holder and is configured to contain a cartridge (23, not shown in figure 2) made of e.g. glass, the cartridge containing the medication. The cartridge has a distal end covered by a membrane (21, not shown in figure 2).

The cap 11, which has a recess (16, not shown in figure 2) is suitable to cover the distal part of the housing 12. The cap 11 is removed from the housing 11 before medication delivery and is attached after medication delivery, wherein the distal part of the housing 11 is inserted to the recess of the cap 11.

Before medication delivery the needle unit 6 with the needle 8 is attached to the needle hub 13 located at the distal end of the housing 12. The needle hub 13 is configured to be releasably coupled with the attachment 7 of the needle unit 6. The needle hub 13 comprises an external thread 14 which matches with the internal thread 10 of the needle unit. The needle unit 8 is attached to the housing 1 so that a proximal end of the needle 8 punctures the membrane of the cartridge. During medication delivery the medication is administered from the cartridge through the needle 8. The needle unit 6 can be detached after medication delivery.

The needle unit 6 is attached to the needle hub 13 by means of the cap 11, which serves as attachment tool. The recess of the cap 11 is configured to hold the outer cover 2 of the needle packaging 1. In this embodiment the recess is formed such that a part 26 of the outer cover 2 protrudes. The user may use the protruding part 26 for inserting and/or removing the outer cover 2. In an alternative embodiment the packaging does not protrude from the recess.

The needle packaging 1 is inserted to the opening of the cap 11 so that the seal 3 is located at the proximal end of the cap 11. Once in place in the cap 11, the sterile seal 3 on the outer cover 2 can be peeled off and the cap 11 is used to locate and attach the needle unit 6 to the needle hub 13.

The cap 11 allows the user to locate the needle unit 6 with its outer cover 2. The user's hand 15 is at a significantly safe distance from the needle 8.

Figure 3 shows the needle unit 6 located inside the cap 11 after the seal 3 has been peeled off. The cap 11 is used as a holding and attachment tool, with the primary benefits being good and safe purchase of the needle unit 6 when picking up and attaching the needle unit 6 to the needle hub 13.

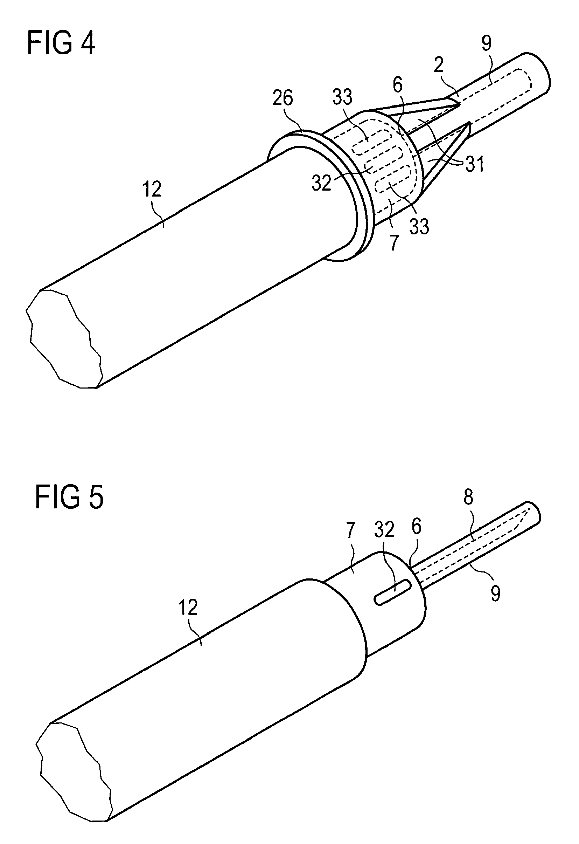

Figure 4 shows the needle unit 6 attached to the needle hub. In this embodiment the outer cover 2 remains on the needle unit 6 after removing the cap 11 from the attached needle unit 6. Before medication delivery, the user removes the outer cover 2 by using his or her hand.

In an alternative embodiment the cap 11 is configured to detach the outer cover 2 from the attached needle unit 6 when the cap 11 is being removed from the needle unit 6. In this case the outer cover 6 may be trapped inside the cap 11 by friction. The outer cover 2 can be removed from the cap 11 by the user. The outer cover 2 can be removed from the apparatus by being shaken out or taken out with one's hand. In the latter case the apparatus is formed such that a part 26 of the outer cover 2 protrudes from the cap 11 if the outer cover 2 is inserted to the cap 11. The user can grip the protruding part 26 for removing the outer cover 2 from the cap 11.

Alternatively the outer cover 2 remains in the cap 11 until the cap 11 is used for detaching the needle unit 6 as described later.

Figure 4 also shows the first, second and third mating means 31, 32, 33. The first mating means 31 may be designed as ribs or webs located on the outside wall of the outer cover 2. The first mating means would interact with the fourth mating means (22, not shown in figure 4), which are located in the cap 11. The fourth mating means, such as ribs or webs, may be moulded within the recess of the cap 11 interacting with the form of the outer cover 2 to transmit the required torque for fitting or removal of the needle unit 6 to the needle hub 13. The fourth mating means of the cap 11 could be designed to interact with only one type of needle cover or a specifically designed needle cover.

The second and third mating means 32, 33 are formed as ribs. The second mating means is formed as rib 32 extending in the axial direction, the rib 32 being arranged on the outer side wall of the attachment 7. The third mating means is formed as ribs 33 extending in the axial direction, the ribs 33 being arranged on the inner side wall of the proximal part 42 of the outer cover. If the needle unit 6 is located inside the outer cover 2, the rib 32 of the second mating means is located between the ribs 33 of the third mating means. In one embodiment at least one of the second and third mating means are designed circumferentially on the needle unit 6 and the outer cover 2, respectively.

Figure 5 shows the attached needle unit 6 after removal of the outer cover 2. The inner cover 9 covers the needle 8 in order to prevent accidental sticking. Shortly before medication delivery the user removes the inner cover 2.

In addition to or separate to the above-mentioned means for holding, attachment and/or detachment, means provided within the cap 11 can be used to locate and store the inner cover 9 of the needle unit 6 between subsequent medication deliveries. In this case the inner cover 9 is reattached between a first and a second medication delivery when the cap 11 is reattached to the medication delivery device after the first medication delivery. The needle unit 6 remains attached between the first and second medication delivery. Before the second medication delivery the inner cover is removed.

Reattaching the inner cover 2 is not limited to cases of using the needle unit 6 twice or repeatedly. The inner cover 2 is preferably reattached to the needle unit 6 before detachment of the needle unit 6 for the purpose of preventing needle sticking injuries when the needle unit 6 is detached and/or disposed.

The cap 11 comprises a holding cavity 20 located at the inside end of the recess 16 which would allow the inner cover 9 to be located centrally on the needle 8. The user could then place the cap 11 over the distal part of the housing of the medication delivery device and the inner cover 9 would align with the needle 8. The distal part of the needle 8 would pick up the inner cover 8. Then the cap 11 could be removed and used to replace the outer cover 2 and completely remove the needle unit 6 from the device for disposal.

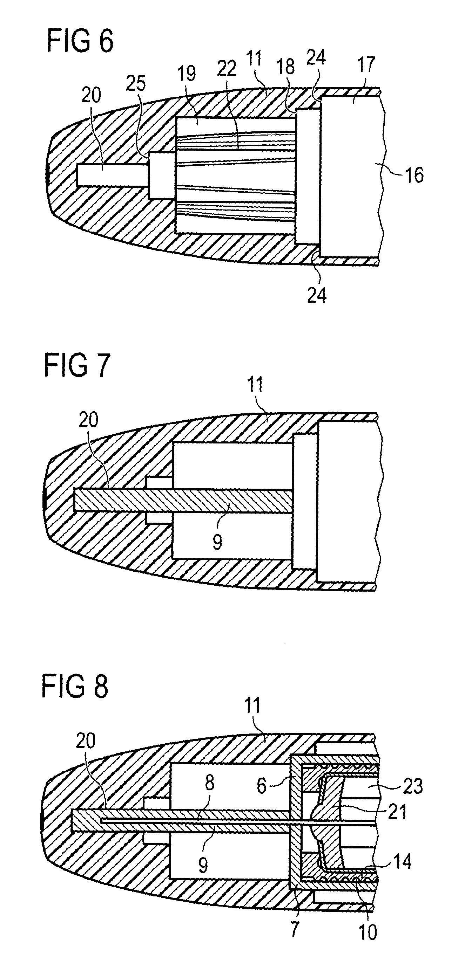

Figure 6 shows a detailed section of the distal part of the cap 11.

The cap 1 comprises a recess 16 suitable to cover the distal part of the housing 12. The recess 16 is also suitable to hold and store the inner cover 9 (not shown in figure 6) before reattaching it and to attach and detach the needle unit 6.

The recess 16 comprises a proximal cavity 17 having a distal wall 18. A distal cavity 19 is located on the distal wall 18 of the proximal cavity.

The proximal cavity 17 is formed such that the needle unit 6 attached to the housing 12 can be inserted into the cap 11 until the distal end of the attachment 7 reaches the distal wall 18 of the proximal cavity.

The recess 16 further comprises means for stopping the distal movement of the outer cover 2, which may be or may not be attached on the needle unit 6. One embodiment of the stopping means is formed as protruding edges 24 of the side wall of the proximal cavity 17. The protruding edges 24 are dimensioned such that the attachment 7 without the outer cover would slide along the protruding edges 24 until the attachment 7 reaches the distal wall 18 of the proximal cavity 17. However, the protruding edges 24 interact with the proximal part of the outer cover 41. The distal movement of the outer cover 2 is stopped when its proximal part 41 reaches the protruding edges 24. Alternatively or additionally the distal movement of the outer cover 2 is stopped when its distal part 42 reaches stopping means located inside the recess. The stopping means may be designed as protruding edges 25 located in the distal cavity 19.

When the outer cover 2 has been stopped, the distal part 42 of the outer cover is located in the distal cavity 19 and interacts with ribs 22, which are arranged on the wall of the distal cavity 19. The ribs 22 serve as fourth mating means which interact with the first mating means 31 of the outer cover 2 so that a rotational movement of the cap 11 is transferred to the outer cover 2.

The inside end of the distal cavity 19 is formed as holding cavity 20. The holding cavity 20 is suitable to hold the inner cover 9 and to position the inner cover 9 such that the needle 8 of the needle unit 6, which is attached to the housing 12, is inserted to the inner cover 9 when the cap 11 is attached to the housing. In one embodiment the holding cavity 20 is formed cylindrically, having a diameter which corresponds to the diameter of the inner cover 9.

A part of the distal cavity 19 located between the proximal recess 17 and the holding cavity 20 serves as guiding part which is designed such that the inner cover 9 slides into the holding cavity 20 when the user inserts the inner cover 9 to the opening of the cap 11. In the embodiment shown in figure 6 the guiding part is formed cylindrically, having a larger diameter than the holding cavity 20 and the inner cover 9. Guiding means formed as ribs 22 are located at the inner wall of the guiding part. The ribs 22 extend in the axial direction and narrow in the distal direction. The ribs 22 also serve as fourth mating means interacting with the first mating means 31 of the outer cover 2. (For clarity reasons the ribs are not shown in figures 7 and 8.) An alternative embodiment of the guiding part (not shown) is formed as truncated cone.

Figure 7 shows the detailed section of the cap 11, wherein the inner cover 9 is held in the predetermined position by the holding cavity 20.

Figure 8 shows a detailed section of the cap 11 holding the inner cover 9, the cap 11 being placed on the distal part of the needle 8.

The needle unit 6 is attached to the needle hub 13, which is releasably connected with the attachment 7 of the needle unit by means of the threads 10, 14. The proximal part of the needle 8 punctures the membrane 21 of the cartridge 23.

After medication delivery the user places the cap 2 on the distal part of the housing 12, to which the needle unit 6 is attached. The cap 11 is formed such that the needle 8 is guided to be aligned with the inner cover 9 held inside the cap 11. When the cap 11 has been attached, the inner cover 2 is attached to the needle 8.

The attached inner cover 9 is connected to the needle unit 6 in a friction-locked manner, so that the cap 11 can be removed from the housing 12 without removal of the inner cover 9.

The cap 11 offers to the user storage for the inner cover 9 and also an alignment and reattachment means for the inner cover 9. This reduces the risk of needle sticking injuries when the user tries to align this small component with the sharp needle 8. The replacement of the inner cover 9 after use reduces the risk of needle sticking injuries by the needle 8 puncturing the side of the outer needle 2 cover when the user replaces the outer cover 2 without using the cap 11.

After reattachment of the inner cover 9 the cap 11 can be used to replace the outer cover 2 and completely remove the needle unit 6 from the medication delivery device for disposal. The outer cover 2 is inserted into the cap 11 which is used to place the outer cover 2 over the attached needle unit 6 and to detach the needle unit 6. In one embodiment the cap 11 is used to unscrew the needle unit 6.

In one embodiment moulded mating means are provided within the end of the cap 11 allowing the cap for transmitting the required torque to remove the needle unit 6 from the needle hub 13. In an alternative embodiment (not shown) the cap is used to disconnect a snapping connection between the needle unit 6 and the needle hub 13. The cap 11 also ensures that, with the exception of the sterile seal 3, the needle unit 6 is disposed of with its original packaging.

### Reference numerals

- 1: needle packaging
- 2: outer cover
- 3: seal
- 4: tap
- 6: needle unit
- 7: attachment
- 8: needle
- 9: inner cover
- 11: cap
- 10: internal thread
- 12: housing
- 13: needle hub
- 14: external thread
- 15: user's hand
- 16: recess
- 17: proximal cavity
- 18: distal wall
- 19: distal cavity
- 20: holding cavity
- 21: membrane
- 22: ribs
- 23: cartridge
- 24, 25: protruding edge
- 26: protruding part of the outer cover
- 31: first mating means
- 32: second mating means
- 33: third mating means
- 41: proximal part of the outer cover
- 42: distal part of the outer cover

## Claims

1. Apparatus comprising a recess (16) configured to hold a cover (2; 9) which is suitable to cover at least a part of a needle unit (6) for a medication delivery device.

2. Apparatus according to claim 1, **characterized in that** the apparatus is configured to attach the cover (2; 9) to the needle unit (6).

3. Apparatus according to claim 1 or 2, **characterized in that** the apparatus comprises holding means (19; 20) configured to encompass at least a part of the cover (2; 9) in a form-fitting manner.

4. Apparatus according to claim 2 or 3, **characterized in that** the apparatus is configured to transmit a transversal and/or a rotational movement of the apparatus to the cover (2; 9).

5. Apparatus according to any of the claims 1 to 4, **characterized in that** the apparatus is configured to attach the needle unit (6), which is at least partly located in the cover (2), to the medication delivery device.

6. Apparatus according to any of the claims 1 to 5, **characterized in that** the apparatus is configured to place the cover (2) over the needle unit (6), which is attached to a medication delivery device, and to detach the needle unit (6) from the medication delivery device.

7. Apparatus according to any of the claims 1 to 4, **characterized in that** the apparatus is configured to attach the cover (9) over a distal part of a needle (8) of the needle unit (6).

8. Apparatus according to claim 7, **characterized in that** the recess (16) is formed to guide the cover to a predetermined position inside the apparatus.

9. Apparatus according to any of the claims 1 to 6, **characterized in that** the recess (16) is suitable to hold the cover which is formed as an outer cover (2) suitable to cover the needle unit (6) with a needle (8) which can be covered by an inner cover (9).

10. Apparatus according to claim 9, **characterized in that** the recess (16) is suitable to hold the outer cover (2) and to hold the inner cover (9).

11. Apparatus according to claim 9, **characterized in that** the recess (16) is suitable to hold one of the outer cover (2) and the inner cover (9), wherein a further recess is suitable to hold the other one of the outer cover (2) and the inner cover (9).

12. Apparatus according to any of the claims 1 to 11, **characterized in that** the apparatus is formed as a cap (11) of the medication delivery device, wherein the cap (11) is attachable to a housing (12) of the medication delivery device so that the cap (11) covers a distal part of the housing (12).

13. Apparatus according to claim 12, **characterized in that** the recess (16) is formed such that the inner cover (9) is attached to the needle (8) when the cap (11) is placed over the distal part of the housing (12).

14. Apparatus according to any of the claims 1 to 13, **characterized in that** the apparatus is suitable to attach and/or detach only one of a multitude of needle units or is suitable to attach and/or detach only a group of a multitude of needle units.

15. Method comprising inserting a cover (2; 9) at least partly into a recess (16) of an apparatus, which is suitable to hold the cover (2; 9) after having been inserted, wherein the cover (2; 9) is suitable to cover at least a part of a needle unit (6) for a medication delivery device.

16. Method according to claim 15, **characterized in that** the cover (2; 9) is attached by the apparatus to the needle unit (6).

17. Method according to claim 15, **characterized in that** the needle unit (6) which is at least partly located in the cover (2) is attached to the medication delivery device by the apparatus.

18. Method according to any of the claims 15 to 17, **characterized in that** the cover (2) is placed over the needle unit (6) by the apparatus, the needle unit (6) being attached to a medication delivery device, and then the needle unit (6) is detached from the medication delivery device.

19. Method according to claim 17 or 18, **characterized in that** the needle unit (6) is screwed to the medication delivery device when the needle unit (6) is being attached and/or the needle unit (6) is unscrewed from the medication delivery device when the needle unit (6) is being detached.

20. Method according to any of the claims 15 to 19, **characterized in that** the cover is formed as an outer cover (2) suitable to cover the needle unit (6) with a needle (8) which can be covered by an inner cover (9).

21. Method according to claim 20, **characterized in that** the apparatus attaches the inner cover (9) to the needle (8).

22. Method according to claim 21, **characterized in that** the inner cover (9) is attached by placing the apparatus over a distal end of a housing (12) of the medication delivery device and then removing the apparatus from the housing (12).

23. Method according to any of the claims 15 to 22, **characterized in that** a cap (11) of the medication delivery device serves as apparatus.

24. Method according to claim 23, **characterized in that** the cap (11) is used to attach and/or detach the needle unit (6).

25. Method according to claim 23 or 24, **characterized in that** the cap (11) is used to attach the inner cover (9).
